# EUROPEAN PATENT APPLICATION

(11) **EP 2 070 518 A2**
(43) Date of publication of application: **17.06.2009**
(21) Application number: 07817380.4
(22) Date of filing: 24.07.2007
(51) Int. Cl.: A61K 9/00, A61K 47/32, A61K 47/36

(54) **OPHTHALMIC SOLUTIONS**

(30) Priority: 25.07.2006 US 833083 P
(71) Applicant: Osmotica Corp., Road Town, Tortola (VG)
(72) Inventor: FAOUR, Joaquina, 1426 Buenos Aires (AR); PASTINI, Ana C., (1416) CA Buenos Aires (AR)
(74) Representative: Weber, Thomas
(86) International application number: PCT/CR2007/000001
(87) International publication number: WO 2008/011836

(57) **Abstract**

Aqueous ophthalmic solutions containing a combination of hyaluronic acid or a pharmaceutically acceptable salt thereof, e.g. sodium hyaluronate, and polyvinyl alcohol are disclosed. These solutions have a synergistic effect on viscosity and provide a statistically significant improvement over the prior art formulations. The solutions are used as artificial tear for the treatment of dry eye syndrome and ocular discomfort and may be administered whenever the use of artificial tears is advisable. The solutions are also suitable for use as vehicle for ophthalmic drugs.

## Description

### FIELD OF THE INVENTION

The present invention relates to ophthalmic solutions. In particular, this invention relates to ophthalmic solutions that contain a combination of polymers that have a synergistic effect on viscosity and provide a statistically significant improvement over the prior art formulations.

### BACKGROUND OF THE INVENTION

Polymeric ingredients are used to increase the viscosity of ophthalmic solutions. Certain combinations of polymers used in ophthalmic solutions are known to provide synergistic effects in viscosity.

U.S. Pregrant Pub. No. 20040253280 to Chowhan et al. discloses various two-polymer combinations reportedly possessing a synergistic effect on viscosity, for example, hydroxypropyl methylcellulose and guar gum, hydroxypropyl methylcellulose and a carboxyvinyl polymer, a carboxyvinyl polymer and guar gum, hydroxypropyl methylcellulose and hydroxyethylcellulose, hyaluronic acid and hydroxypropyl methylcellulose, and hyaluronic acid and guar gum. However, various two-polymer combinations were disclosed as lacking a synergistic effect on viscosity, for example: polyvinyl alcohol and chondroitin sulfate; polyvinyl alcohol and polyvinylpyrrolidone; chondroitin sulfate and polyvinylpyrrolidone; polyvinyl alcohol and carbomer; chondroitin sulfate and carbomer; polyvinylpyrrolidone and carbomer; hydroxypropyl methylcellulose and dextran; guar and dextran; and carbomer and dextran.

In a similar fashion, Chowhan et al. (U.S. Pregrant Pub. No. 20040253202) disclose three-polymer combinations reportedly possessing a synergistic effect on viscosity, for example hydroxypropyl methylcellulose (HPMC), guar gum and a carboxyvinyl polymer; HPMC, guar gum and hydroxyethyl cellulose; HPMC, guar gum and dextran; HPMC, hydroxyethyl cellulose and a carboxyvinyl polymer; and HPMC, dextran and a carboxyvinyl polymer. However, various three-polymer combinations were disclosed as lacking a synergistic effect on viscosity, for example, polyvinyl alcohol, chondroitin sulfate and polyvinylpyrrolidone; polyvinyl alcohol, chondroitin sulfate, and carbomer; polyvinyl alcohol, polyvinylpyrrolidone, and carbomer; and chondroitin sulfate, polyvinylpyrrolidone, and carbomer.

Hyaluronan is a mucopolysaccharide that occurs naturally in the skin, synovial fluid, and vitreous humour of the eye of humans and other animals. The term hyaluronan encompasses hyaluronic acid as well as salts of hyaluronic acid, such as sodium hyaluronate. The repeating disaccharide unit of hyaluronan consists of alternating glucuronic acid and N-acetylglucosamine units, which are repeated over and over to form long chains. Each repeating disaccharide unit has one carboxylate group, four hydroxyl groups, and an acetamido group. Hyaluronan belongs to a group of polysaccharides known as glycosaminoglycans. Several ophthalmic solutions for the treatment of dry eye and/or eye discomfort have been formulated to contain sodium hyaluronate, e.g. Dropstar^{®} TG (Farmigea, Italy), Vismed^{®} (Chemedica, Switzerland), Vislube^{®} (Thea, US; Chemedica, Switzerland), Hyasol (manufacture by Lab. Pablo Cassará S.R.L. for Bausch & Lomb Argentina S.R.L.), Hyalistil (Sifi, Italy), Ialurex Ipotonico (Fidia, Italy), Hialudorf (PharmaDorf, Argentina), Oxyal (Santen, Germany), Lacripharma (ICN Argentina), Dunason^{MR} which also contains condroitin sulfate as active principle (Alcon Laboratórios Do Brasil, Ltd.), and Maxus which also contains condroitin sulfate as active principle (Bausch & Lomb Argentina). Panoptic Lagrimas (Bausch & Lomb Argentina) is formulated to contain polyvinyl alcohol (0.5% w/v), povidone (0.6% w/v), potassium chloride (0.12% w/v), sodium chloride (0.8% w/v), potassium sorbate (0.18% w/v), and sodium hyaluronate (0.15% w/v).

Hyaluronate eye drops are reportedly useful for treating severe dry eye in patients with Sjogren's syndrome (Br. J. Ophthalmol. (2002 Aug), 86(8), 879-84).

European Patent Application Publication EP0323522 to Iwao et al. discloses an artificial tear and therapeutic agent for corneal xerosis containing sodium hyaluronate which is useful as a tear supplement. The composition essentially contains sodium hyaluronate, sodium chloride, preservatives, and buffers.

U.S. Pat. No. 5,770,628 to Cantoro discloses an ophthalmic preparation for use as an artificial tear containing hyaluronate as a viscosity thickener, preferably in the form of sodic salt and having a molecular weight of 500,000 to 4,000,000 daltons, at a concentration of 0.05 to 2% by weight, as well as the following minimum quantities of ionic species: 40 mmol/l sodium ion, 12 mmol/l potassium ion, 0.4 mmol/l calcium ion, 0.4 mmol/l magnesium ion, 50 mmol/l chloride ion, 7 mmol/l phosphate ion and, preferably, 0.7 mmol/l citrate ion.

U.S. Pat. No. 6,528,465 to Cantoro discloses an ophthalmic solution with viscosity-enhancing and detergent properties for contact lenses. The solution contains one or more physiologically acceptable viscosity enhancing agents in an aqueous solution having a non-Newtonian rheological behavior, and one or more physiologically acceptable non-ionic surfactants. The viscosity enhancing agent is hyaluronic acid or its salts with alkali or alkaline earth metals, and the non-ionic surfactant is poloxamer.

U.S. Pregrant Pub. No. 20050152951 to Lloyd discloses a liquid, eye-instillable preparation comprising a viscosity-enhancing agent which can be one or both of sodium hyaluronate and chondroitin sulphate, a preservative (polyhexanide), and one or more carriers in which the agent and the preservative are dispersed.

U.S. Pregrant Pubs. No. 20040137079, No. 20050260280, and No. 20050266089, to Cook et al. disclose a stable ophthalmic composition which is reportedly comfortable to the human eye comprising. The composition contains about 0.1% to about 0.6% w/v hyaluronic acid; and about 0.0020% to about 0.02% w/v stabilized oxy-chloro complex. The composition can also contain a polyol demulcent such as glycerin, polyethylene glycol 300, polyethylene glycol 400, polysorbate 80 and propylene glycol, or a cellulose derivative demulcent such as carboxymethylcellulose sodium, hydroxyethyl cellulose, hydroxypropyl methylcellulose, and methylcellulose.

U.S. Pregrant Pub. No. 20060008443 to Chang et al. discloses ophthalmic compositions containing an acceptable carrier component, for example, an aqueous-based carrier, and a plurality of polyanionic component portions having different molecular weights. A particularly useful class of polyanionic components are one or more polymeric materials having multiple anionic charges such as hyaluronic acid.

U.S. Pregrant Pub. No. 20060029571 to Karageozian et al. discloses stabilized hyaluronan preparations wherein hyaluronan is combined with a polyglycol, such as polyethylene glycol.

PCT International Application Publication No. WO 1991/12808 to Hills discloses an artificial tear composition for use in treating or preventing dry eye syndrome, or sore eyes. The solutions contains a phospholipid, and optionally hyaluronic acid or its salts, in a suitable carrier. The carrier is preferably an isotonic salt solution such as saline, or else propylene glycol.

PCT International Application Publication No. WO 2003/011305 to Babiole Saunier et al. discloses an ophthalmic pharmaceutical composition for treatment of dry eye symptoms. The solution contains a hyaluronate and hydrogen peroxide.

PCT International Application Publication No. WO 2003/049747 to Gross et al. discloses a pharmaceutical composition that contains at least panthenol and/or pantothenic acid and hyaluronic acid and/or hyaluronate and optionally pharmaceutical adjuvants.

RU2163123 to Aznabaev et al. discloses ophthalmic drops containing high-molecular hyaluronic acid of 95% purity isolated from human umbilical cord as a biologically active substance and physiological solution.

JP4069342 to Ushio et al. discloses a stable aqueous medicinal preparation that possesses antiseptic properties and contains benzalkonium chloride and boric acid as a preservative in an aqueous solution of hyaluronic acid.

JP5320055 to Mitsuno et al. discloses an allergy therapeutic agent not having any adverse action but having an excellent antiallergic action different from those of conventional allergy-therapeutic agents. This allergy therapeutic agent contains hyaluronic acid and/or its nontoxic salt such as sodium salt, potassium salt or calcium salt as an active ingredient.

JP2002020279 to Egami discloses an eye lotion for treating ectocornea disorder. The eye lotion contains 0.05-0.3% w/v; hyaluronic acid or salt thereof, and 1.0-3.0% w/v taurine.

JP2002255829 to Okada et al. discloses a collyrium solution composition having a tear layer-protecting effect and containing hyaluronic acid and/or its salt.

JP2005187354 to Odaka discloses an aqueous external preparation composition, especially an ophthalmic composition, which contains one or more compounds selected from hyaluronic acid or its salts, aminoethylsulfonic acid, L-aspartic acid, epsilon-aminocaproic acid, chondroitin sulfate, and their salts. The solution reportedly has excellent antiseptic properties and does not irritate eyes or nasal mucosa. The solution contains an alcohol derivative as an antiseptic agent and a liquid containing hyaluronic acid or its salt, and one or more compounds selected from L-aspartate, epsilon-aminocaproic acid, and chondroitin sulfate.

None of the prior art ophthalmic solutions disclose a combination containing hyaluronic acid or a pharmaceutically acceptable salt thereof, e.g. sodium hyaluronate, as an active ingredient and polyvinyl alcohol having a synergistic effect on viscosity that provides long retention times and contributes considerably to lessen the epithelial surface damage.

### SUMMARY OF THE INVENTION

It is an object of the invention to provide ophthalmic solutions, suitable for ophthalmic administration that overcome the disadvantages of and provide a statistically significant improvement over the prior art formulations.

The present invention provides ophthalmic solutions containing a combination of hyaluronic acid or a pharmaceutically acceptable salt thereof, e.g. sodium hyaluronate, and polyvinyl alcohol. The solutions possess an enhanced viscosity and lubricating property and provide a statistically significant improvement over the prior art formulations.

Another object of the invention is to provide ophthalmic solutions that are easily administrable in drop form and have an appreciable duration of action.

The invention provides an aqueous ophthalmic solution comprising a viscosity enhancing amount of the combination of 0.05 to 0.4 % w/v hyaluronic acid or a pharmaceutically acceptable salt thereof, 0.25 to 4.0 % w/v polyvinyl alcohol, and a viscosity of 20 to 150 centistoke.

The invention also provides an aqueous ophthalmic solution comprising a viscosity-enhancing amount of the combination of 0.05 to 0.4 % w/v hyaluronic acid or a pharmaceutically acceptable salt thereof, and 0.25 to 4.0 % w/v polyvinyl alcohol, wherein the solution has less than 0.6 % w/v the one or more ionic-tonicity agents and a viscosity of 20 to 150 centistoke.

It is another object of the invention to provide an ophthalmic solution that is useful as an artificial tear and is effective in the treatment of dry eye syndrome and ocular discomfort.

The ophthalmic solutions of the invention are useful for treating rhinological allergic complications. The invention also provides a method for treating conditions selected from the group consisting of dry eye syndrome, keratitis sicca, xerophthalmia, keratoconjunctivitis sicca, ocular discomfort, rhinological allergic complications, Sjogren's syndrome, and Stevens Johnson syndrome comprising the step of administering an ophthalmic solution of the invention.

The invention also provides ophthalmic solutions which are useful as a platform to deliver active agents such as 1) anti-glaucoma agents, 2) anti-infective agents, 3) antiinflammatory agents, 4) antihistamines 5) anti-allergic agents, 6) decongestants, 7) hormones, 8) vitamins, 9) growth factors, 10) cytokines, 11) mucins, 12) surface stimulating drugs, 13) immunomodulators, 14) immune response modifiers, 15) cytokine modifying agents, 16) immunosuppressive agents, 17) antineoplastic agents, 18) anti-angiogenesis agents, 19) eyelash growth stimulators and other medicaments, and mixtures thereof.

Some embodiments of the invention include those wherein: 1) the anti-glaucoma agent is a beta-blocker 2) the beta-blocker is selected from the group consisting of timolol, betaxolol, levobetaxolol, carteolol, metipranolol, levobunolol, 3) the anti-glaucoma agent is a miotic, 4) the miotic is pilocarpine, 5) the anti-glaucoma agent is a carbonic anhydrase inhibitor, 6) the carbonic anhydrase inhibitor is selected from the group consisting of dorzolamide, brinzolamide, 7) the anti-glaucoma agent is a prostaglandin, 8) the prostaglandin is selected from the group consisting of travoprost, latanoprost, bimatoprost, 9) the anti-glaucoma agent is a serotonergic, 10) the anti-glaucoma agent is a muscarinic, 11) the anti-glaucoma agent is a dopaminergic agonist, 12) the anti-glaucoma agent is an adrenergic agonist, 13) the adrenergic agonist is selected from the group consisting of apraclonidine, brimonidine, dipivefrin, 14) the anti-infective agent is an antibiotic, 15) the antibiotic is selected from the group consisting ciprofloxacin, ofloxacin, moxifloxacin, tobramycin, gentamicin, sulfacetamide, trimethoprim, polymyxin B, 16) the anti-infective agent is an antimicrobial peptide, 17) the anti-infective agent is an antiviral, 18) the antiviral is trifluridine, 19) the anti-infective agent is an antiparasitic, 20) the anti-infective agent is an antifungal, 21) the anti-inflammatory agent is a non-steroidal anti-inflammatory agent, 22) the non-steroidal steroidal anti-inflammatory agent is selected from the group consisting of suprofen, flurbiprofen, diclofenac, ketorolac, 23) the anti-inflammatory agent is a steroidal anti-inflammatory agent, 24) the steroidal anti-inflammatory agent is selected from the group consisting of rimexolone, tetrahydrocortisol, prednisolone, dexamethasone, 25) the anti-allergic agent is selected from the group consisting of olopatadine, cromolyn, lodoxamide, emedastine, epinastine, 26) the decongestant is selected from the group consisting of naphazoline, phenylephrine, 27) the hormone is an androgen, 28) the growth factor is epidermal growth factor (EGF).

Other features, advantages and embodiments of the invention will become apparent to those skilled in the art by the following description, accompanying examples.

### DETAILED DESCRIPTION OF THE INVENTION

The aqueous ophthalmic solutions of this invention can be directly applied onto the ocular surface of a patient. The solutions comprise a combination of hyaluronic acid or a pharmaceutically acceptable salt thereof, e.g. sodium hyaluronate, and polyvinyl alcohol (PVA). Together, the hyaluronan, e.g. sodium hyaluronic acid, and PVA provide a synergistic effect on viscosity.

The compositions of the invention comprise hyaluronan. The term hyaluronan encompasses hyaluronic acid as well as salts of hyaluronic acid, such as sodium hyaluronate. Sodium hyaluronate (NaHA, sodium hyaluronic acid) is a highly lubricious, hydrophilic polymer that provides a protective effect against eye dryness. Genzyme Corp. (Cambridge, MA) and Hyaluron Inc. (Burlington, MA), among other companies, sell sodium hyaluronate with molecular weights ranging from 0.5 to greater than 3.0 million Dalton. NaHA is described in the Merck Index, 11th edition (Merck Index No.: 12, 4793) and is monographed in the European Pharmacopoeia, 3rd Edition, (Supplement 2000, pages 1190-1193, Appendix 3). The CAS Registry No. for sodium hyaluronate is 9067-32-7. Some approximate average molecular weight ranges for hyaluronic acid include 200,000 to 4,000,000 Daltons, 750,000 to 2,000,000 Daltons, 800,000 to 1,750,000 Daltons, 900,000 to 1,500,000 Daltons, or 1,000,000 Daltons.

The content of hyaluronic acid or a pharmaceutically acceptable salt thereof, e.g. sodium hyaluronate, in the ophthalmic solutions of this invention preferably ranges from 0.05 to 0.4 or from 0.05 to 0.3 or from 0.1 to 0.3 or about 0.2 % (w/v).

Polyvinyl alcohol (PVA) is a water soluble polymer produced by polymerization of vinyl acetate followed by hydrolysis of the polyvinyl acetate polymer. The degree of polymerization determines the molecular weight and viscosity in solution. PVA can be obtained from companies such as Jiangsu Dongzhan Chemical Co., Ltd (China). The content of polyvinyl alcohol in the ophthalmic solutions of this invention preferably ranges from 0.25 to 4.0 or 0.25 to 3 % (w/v).

The synergistic effect on viscosity provided by the combination of sodium hyaluronate and polyvinyl alcohol is disclosed in Examples 1, 2 and 3. Solution A of Example 1 comprises sodium hyaluronate (0.1% w/v), polyvinyl alcohol (2.0% w/v), an humectant agent (1.0% w/v ), a lubrication agent (0.4% w/v), a tonicity agent (0.38% w/v), a buffering agent (0.2% w/v + 0.05% w/v) and a preservative agent (0.001% w/v). Solution B of Example 1 comprises the same components of solution A except sodium hyaluronate. Solution C of Example 1 comprises the same components of solution A except polyvinyl alcohol. The viscosity of solution A, 20.19 cSt, is greater than 90% of the simple sum of the viscosity of solution B, 6.59 cSt, and C, 3.74 cSt. Solution D of Example 2 comprises sodium hyaluronate (0.2% w/v), polyvinyl alcohol (2.0% w/v), an humectant agent (1.0% w/v), a lubrication agent (0.4% w/v), a buffering agent (0.2% w/v + 0.05% w/v) and a preservative agent (0.01% w/v). Solution E of Example 2 comprises the same components of solution D except sodium hyaluronate. Solution F of Example 2 comprises the same components of solution D except polyvinyl alcohol. The viscosity of solution D, 74.50 cSt, is greater than 140% of the simple sum of the viscosity of solution E, 6.81 cSt, and F, 23.57 cSt. Solution G of Example 3 comprises sodium hyaluronate (0.2% w/v), polyvinyl alcohol (2.0% w/v), an humectant agent (1.0% w/v ), a lubrication agent (0.4% w/v), a tonicity agent (0.1% w/v), a buffering agent (0.05% w/v) and a preservative agent (0.18% w/v). Solution H of Example 3 comprises the same components of solution G except sodium hyaluronate. Solution I of Example 3 comprises the same components of solution G except polyvinyl alcohol. The viscosity of solution G, 64.42 cSt, is greater than 105% of the simple sum of the viscosity of solution H, 7.16 cSt, and I, 23.44 cSt.

The ophthalmic solution Panoptic Lagrimas (Bausch & Lomb Argentina) is formulated to contain polyvinyl alcohol (0.5% w/v), povidone (0.6% w/v), potassium chloride (0.12% w/v), sodium chloride (0.8% w/v), potassium sorbate (0.18% w/v), and sodium hyaluronate (0.15% w/v), and has an average viscosity of 9.67 cSt, an average osmolality of 331 mOsm/kg, and an average pH value of 6.09 (all measured three times). Even though the Panoptic Lagrimas comprises a combination of sodium hyaluronate and polyvinyl alcohol, such solution has a viscosity of 9.67 cSt compared to 74.50 cSt of the solution D of the invention. The high viscosity of the solutions of the present invention would result in long retention time and contribute considerably to lessen the epithelial surface damage and therefore would be advantageous.

The solution may include one or more pharmaceutically acceptable buffering agents, preservatives (including preservative adjuncts), tonicity-adjusting agents, surfactants, solubilizing agents, stabilizing agents, comfort-enhancing agents, emollients, pH-adjusting agents, demulcents and/or lubricants.

Humectant (demulcent) agents which can be added to the solution of the invention are selected from the group comprising glycerin, propylene glycol, hydroxypropyl methyl cellulose, carboxymethyl cellulose, methyl cellulose, hydroxyethyl cellulose, polyvinyl pyrrolidone, polyethylene oxide, polyethylene glycol and polyacrylic acid, and mixtures thereof. The humectant agents are used in effective amounts to lubricate mucous membrane surfaces and to relieve dryness and irritation. For example, propylene glycol is used from about 0.2 to about 1.5%, but preferably about 1% (w/w).

A lubricant can be added to the solution of the invention. Exemplary, non-limiting compounds are selected from the group consisting of polyethylene glycol, cellulose derivatives such as carboxymethylcellulose sodium, hydroxyethyl cellulose, hydroxypropyl methylcellulose, and methylcellulose. Other examples of lubricants include dextran 70, gelatin, glycerin, polysorbate 80, propylene glycol, povidone, and mixtures thereof.

Suitable tonicity adjusting agents that may be employed in ophthalmic compositions of the invention include by way of example and without limitation, one or more inorganic salts, electrolytes, sodium chloride, potassium chloride, sodium phosphate, potassium phosphate, sodium, potassium sulfates, sodium and potassium bicarbonates and alkaline earth metal salts, such as alkaline earth metal inorganic salts, e.g., calcium salts, and magnesium salts, mannitol, dextrose, glycerin, propylene glycol, and mixtures thereof. The composition may be adapted with a variety of different osmolalities: iso-osmolal (with respect to the fluids of the eye) solutions which osmolalities typically range from about 175 to about 330 mOsm/kg, but more preferably from about 280 to about 320 mOsm/kg; hypotonic solutions, i.e. around 130-150 mOsm/kg. Increasing the amount of ionic tonicity-adjusting agents, such as sodium chloride, has the effect of decreasing the viscosity of the solutions of the invention.

Buffering agents include by way of example and without limitation, boric acid, dibasic sodium phosphate, monobasic sodium phosphate, hydrochloric acid, sodium hydroxide, tris(hydroxymethyl)aminomethane, bis(2-hydroxyethyl)iminotris-(hydroxymethyl)methane, and sodium hydrogen carbonate and others known to those of ordinary skill in the art, which can be used to adjust the pH. Buffers are commonly used to adjust the pH to a desirable range for ophthalmic use. Usually a pH of around 5 to 9, 5 to 8, 6 to 7.4, 6.5 to 7.5, or 6.9 to 7.4 is desired, however, this may need to be adjusted due to other factors such as the stability or solubility of the therapeutically active agent or other excipients.

Preservative agents are selected from the group comprising benzalkonium chloride, per-salts, such as perborates, percarbonates and the like; alcohols, such as benzyl alcohol, chlorbutanol and the like; peroxides, such as very low concentrations, e.g., about 50 to about 200 ppm (w/v), of hydrogen peroxide and the like; preservative agents containing quaternary ammonium salts such as polyquarterium; biguanide-containing preservatives such as polyhexamethylene biguanide (available from Arch, as Cosmocil^{™} CQ); chlorine dioxide precursors which include stabilized chlorine dioxide (SCD), metal chlorites, such as alkali metal and alkaline earth metal chlorites, and the like; sorbic acid and ophthalmically acceptable salts thereof such potassium sorbate, and mixtures thereof. The amount of preservative agents included in the present solutions containing such a component varies over a relatively wide range depending on the specific preservative agent employed. The amount of such agents preferably is in the range of about 0.000001 % to about 0.05% w/v or more of the compositions of the invention. Benzalkonium chloride is typically used in concentrations from about 0.01 to about 0.10% (w/w). Potassium sorbate is typically used in concentrations from about 0.10 to about 0.20% (w/w). If the preservatives are not added to the ophthalmic solution, the ophthalmic solution can be used as single dose type eye drops, in which the ophthalmic solution is used off in one administration. Otherwise, the ophthalmic solution can be used as multi dose type eye drops included in a container provided with a filter attached to a nozzle of the container, for dispensing the eye drops.

Suitable exemplary ophthalmic demulcents are described in the United States Ophthalmic Demulcents Monograph (See 21 CFR 349.12 (2003)). Suitable additional demulcents include, but are not limited to, cellulose derivatives ranging from about 0.2 to about 2.5 % such as carboxymethylcellulose sodium, hydroxyethyl cellulose, hydroxypropyl methylcellulose, and methylcellulose; gelatin at about 0.01%; polyols in about 0.05 to about 1%, also including about 0.2 to about 1%, such as glycerin, polyethylene glycol 300, polyethylene glycol 400, polysorbate 80, and propylene glycol; polyvinyl alcohol from about 0.1 to about 4 %; povidone from about 0.1 to about 2%; and dextran 70 from about 0.1% when used with another polymeric demulcent described herein.

The ophthalmic solutions of the invention can also be used as a platform to deliver active agents. Active ingredients that could be used include 1) anti-glaucoma agents such as a) beta-blockers including timolol, betaxolol, levobetaxolol, carteolol, metipranolol, levobunolol, b) miotics including pilocarpine, c) carbonic anhydrase inhibitors including dorzolamide, brinzolamide, d) prostaglandins including travoprost, latanoprost, bimatoprost e) serotonergics, f) muscarinics, g) dopaminergic agonists, h) adrenergic agonists including apraclonidine, brimonidine, dipivefrin; 2) anti-infective agents such as a) antibiotics including ciprofloxacin, ofloxacin, moxifloxacin, tobramycin, gentamicin, sulfacetamide, trimethoprim, polymyxin B, b) antimicrobial peptides, c) antivirals including trifluridine, d) antiparasitics, e) antifungals; 3) anti-inflammatory agents such as a) non-steroidal steroidal anti-inflammatory agents including suprofen, flurbiprofen, diclofenac, ketorolac, b) steroidal anti-inflammatory agents including rimexolone, tetrahydrocortisol, prednisolone, dexamethasone; 4) antihistamines 5) anti-allergic agents including, olopatadine, cromolyn, lodoxamide, emedastine, epinastine, 6) decongestants including naphazoline, phenylephrine, 7) hormones such as androgens and others, 8) vitamins, 9) growth factors including epidermal growth factor (EGF), 10) cytokines, 11) mucins, 12) surface stimulating drugs, 13) immunomodulators, 14) immune response modifiers, 15) cytokine modifying agents, 16) immunosuppressive agents, 17) antineoplastic agents, 18) anti-angiogenesis agents, 19) eyelash growth stimulators and other medicaments, and mixtures thereof.

The active agents can be present in its neutral, ionic, salt, basic, acidic, natural, synthetic, diastereomeric, isomeric, enantiomerically pure, racemic, hydrate, solvate, chelate, complex, derivative, analog, pro-drug, amorphous, polymorphous, crude forms, crystalline forms, or other common forms. Unless otherwise specified, when a drug is referred to by name such reference includes all known forms of the drug.

The amount of drug included in the compositions of the present invention will be whatever amount is therapeutically effective and will depend upon a number of factors, including the identity and potency of the chosen drug, the disorder being treated, the health of the subject being treated and other such factors common to the pharmaceutical industry for prescription of drugs to a subject. The drugs will generally be administered according to their known dosing regimens such as those disclosed in the Pharmaceutical Desk Reference or those recognized as suitable by the Food and Drug Administration (USA), European Medicines Agency (Europe), National Institute of Health Sciences (Japan), and National Administration of Drugs, Food, and Medical Technology (Administración Nacional de Medicamentos, Alimentos y Tecnologia Médica, Argentina).

The ophthalmic solutions of the invention can be used for the treatment of dry eye syndrome, also referred to as keratitis sicca, xerophthalmia and keratoconjunctivitis sicca (KCS), ocular discomfort, and rhinological allergic complications such as allergic conjunctival inflammation. The solutions may be administered whenever the use of artificial tears is advisable.

A non randomized, double-masked, observational clinical trial is carried out in humans to compare formulation G of Example 3 with Panoptic Lagrimas (Bausch & Lomb Argentina) according to Example 4. The administration of the formulation of the invention is expected to provide statistical significant longer tear stability measured by the non-invasive break-up time (NIBUT), significantly improved symptoms such as ocular comfort, ocular sensitivity, red eye, blurred vision, itching, and foreign body symptoms as judged by the dry eye questionnaire, statistical significant reduction of the damage of the corneal and/or conjunctival surface as measured by the fluorescein staining, statistical significant reduction of the staining of the ocular surface as measured by the lissamine green staining, and/or a statistical significant improvement of the visual acuity as measure by the visual acuity test.

Patients showed a statistically significant improved Schirmer's test from baseline to the end of treatment with formulation G (p=0.008). The lissamine green staining showed a statistically significant improvement from baseline for both treatment groups at day 30 visit, and a statistically significant difference at day 30 visit favoring formulation G over Panoptic Lagrimas (p=0.043). Improvements in NIBUT were observed with formulation G compared to Panoptic Lagrimas, but a statistically significant difference was not obtained. A statistically significant difference was approached but not reached for the fluorescein staining at the end of treatment (p= 0.063). The Texas Eye Research and Technology Center dry eye symptoms questionnaire (TERTC-DEQ) between treatments showed a better tendency for symptomatology relief with formulation G. Safety data demonstrated that both, formulation G of Example 3 and Panoptic Lagrimas, were safe and well tolerated by dry eye subjects.

The preparations of this invention may be liquid solutions, gels, creams, or any other usable forms. The preparations of this invention may be used for a variety of medical and non-medical (e.g., household or industrial) applications, including topical administration to the eye (e.g., to moisturize the eye, treat dry eye, promote corneal healing, facilitate reepithelialization for non-infectious corneal erosion, management of dry eye syndrome, allergic conjunctivitis, and contact lens wear, etc.), topical administration (e.g., to moisturize the skin, to treat dry skin or dermatological disorders), lubrication of body tissues or body orifices, lubrication of devices (e.g., catheters, scopes, instruments, etc.), application to tissues during surgery to deter post-surgical adhesion formation, etc.

The preparations of the invention can be used for treating ocular surface diseases such as dry eye syndrome where dry eye syndrome ranges from mere sensation, e.g. itchy, scratchy, gritty, tired, red, burning and watery, to pathological dry eye, e.g. Sjogren's syndrome, keratoconjunctivitis sicca, and Stevens Johnson syndrome.

Dorzolamide hydrochloride, a carbonic anhydrase inhibitor, is indicated for the treatment of elevated intraocular pressure in patients with ocular hypertension or open-angle glaucoma. The chemical name of dorzolamide hydrochloride is (4S-trans)-4-(ethylamino)-5,6-dihydro-6-methyl-4H-thieno[2,3-b]thiopiran-2-sulfonamide 7,7-dioxide monohydrochloride. Solution J of Example 5 describes an ophthalmic solution comprising dorzolamide hydrochloride and a viscosity enhancing amount of the combination of sodium hyaluronate and PVA. Solution J comprises dorzolamide hydrochloride (2.23% w/v), sodium hyaluronate (0.2% w/v), polyvinyl alcohol (2.0% w/v), an humectant agent (1.0% w/v), a lubrication agent (0.4% w/v), a tonicity agent (0.1% w/v), a buffering agent (0.05% w/v) and a preservative agent (0.18% w/v). Solution K of Example 5 comprises the same components of solution J except sodium hyaluronate. Solution L of Example 5 comprises the same components of solution J except polyvinyl alcohol. The viscosity of solution J, 53.62 cSt, is greater than 150% of the simple sum of the viscosity of solution K, 6.73 cSt, and L, 14.10 cSt.

The following examples should not be considered exhaustive, but merely illustrative of only a few of the many embodiments contemplated by the present invention. The methods described herein can be followed to prepare ophthalmic solutions according to the invention.

### EXAMPLE 1

Ophthalmic solutions were prepared as described below containing the following ingredients in the amounts indicated in Table 1:

| **Table 1** | | | |
|---|---|---|---|
| **Ingredient** | **Composition (%w/v)** | | |
| | **A** | **B** | **C** |
| PVA | 2.0 | 2.0 | --- |
| Sodium hyaluronate | 0.1 1 | --- | 0.1 |
| NaH₂PO₄.H₂O | 0.05 | 0.05 | 0.05 |
| Na₂HPO₄.12H₂O | 0.2 | 0.2 | 0.2 |
| Propylene glycol | 1.0 | 1.0 | 1.0 |
| Polyethylene glycol 400 | 0.4 | 0.4 | 0.4 |
| NaCl | 0.38 | 0.38 | 0.38 |
| Cosmocil CQ | 0.001 | 0.001 | 0.001 |
| NaOH | q.s. pH 7.4 | q.s. pH 7.4 | q.s. pH 7.4 |
| Water | q.s. to 100 ml | | |

Polyvinyl alcohol was slowly added to purified water, using around 80% of the desired batch volume, and allowed to hydrate during approximately 5 hours with continuous stirring. Then, the mixture was heated to 80°C with continuous mixing. Next, the solution was cooled to room temperature (between 22-25°C) with constant stirring. Then, the following ingredients were added slowly at room temperature with continuous mixing, waiting until each ingredient was dissolved before adding the next: sodium hyaluronate, NaH₂PO₄.H₂O Na₂HPO₄.12H₂O, propylene glycol, polyethylene glycol 400, NaCl and finally Cosmocil CQ. The pH was then adjusted with a 10% w/v solution of NaOH, and the remaining amount of purified water was added. The solutions were independently prepared 3 times each, and the pH, osmolality, density, and viscosity were measured. The averages of the results for each solution are shown in the table below.

| **Parameter** | **Composition** | | |
|---|---|---|---|
| | **A** | **B** | **C** |
| **pH value** | 7.44 | 7.39 | 7.46 |
| **Osmolality (mOsm/kg)** | 338 | 327 | 308 |
| **Density (g/ml)** | 1.007 | 1.011 | 1.005 |
| **Viscosity (cSt)*** | 20.19 | 6.59 | 3.74 |

| | | | |
|---|---|---|---|
| *Viscosity values where measured with Cannon Fenske Routine Type Viscometers, size 100 and 150 (measuring ranges: 3-15 and 7-35 cSt, respectively). | | | |

### EXAMPLE 2

An ophthalmic solution was prepared as described below containing the following ingredients in the amounts indicated in Table 2:

| **Table 2** | | | |
|---|---|---|---|
| **Ingredient** | **Composition (%w/v)** | | |
| | **D** | **E** | **F** |
| PVA | 2.0 | 2.0 | --- |
| Sodium hyaluronate | 0.2 | --- | 0.2 |
| NaH₂PO₄.H₂O | 0.05 | 0.05 | 0.05 |
| Na₂HPO₄.12H₂O | 0.2 | 0.2 | 0.2 |
| Propylene glycol | 1.0 | 1.0 | 1.0 |
| Polyethylene glycol 400 | 0.4 | 0.4 | 0.4 |
| Benzalkonium Chloride | 0.01 | 0.01 | 0.01 |
| NaOH | q.s. pH 7.4 | q.s. pH 7.4 | q.s. pH 7.4 |
| Water | q.s. to 100 ml | | |

Polyvinyl alcohol was slowly added to purified water, using around 80% of the desired batch volume, and allowed to hydrate during approximately 5 hours with continuous stirring. Then, the mixture was heated to 80°C with continuous mixing. Next, the solution was cooled to room temperature (between 22-25°C) with constant stirring. Then, the following ingredients were added slowly at room temperature with continuous mixing, waiting until each ingredient was dissolved before adding the next: sodium hyaluronate, NaH₂PO₄.H₂O, Na₂HPO₄.12H₂O, propylene glycol, polyethylene glycol 400, and finally benzalkonium chloride. The pH was then adjusted with a 10% w/v solution of NaOH, and the remaining amount of purified water was added. The solutions were independently prepared 3 times each, and the pH, osmolality, density, and viscosity were measured. The averages of the results for each solution are shown in the table below.

| **Parameter** | **Composition** | | |
|---|---|---|---|
| | **D** | **E** | **F** |
| **pH value** | 7.41 | 7.41 | 7.39 |
| **Osmolality (mOsm/kg)** | 213 | 195 | 178 |
| **Density (g/ml)** | 1.008 | 1.007 | 1.003 |
| **Viscosity (cSt)**** | 74.50 | 6.81 | 23.57 |

| | | | |
|---|---|---|---|
| **Viscosity values where measured with Cannon Fenske Routine Type Viscometers, sizes 100, 150 and 200 (measuring ranges: 3-15 cSt, 7-35 cSt, and 20-100 cSt, respectively). | | | |

### EXAMPLE 3

An ophthalmic solution was prepared as described below containing the following ingredients in the amounts indicated in Table 3:

| **Table 3** | | | |
|---|---|---|---|
| **Ingredient** | **Composition (%w/v)** | | |
| | **G** | **H** | **I** |
| PVA | 2.0 | 2.0 | --- |
| Sodium hyaluronate | 0.2 | --- | 0.2 |
| NaH₂PO₄.H₂O | 0.05 | 0.05 | 0.05 |
| Propylene glycol | 1.0 | 1.0 | 1.0 |
| Polyethylene glycol 400 | 0.4 | 0.4 | 0.4 |
| NaCl | 0.1 | 0.1 | 0.1 |
| Potassium sorbate | 0.18 | 0.18 | 0.18 |
| HCl | q.s. pH 5.5 | q.s. pH 5.5 | q.s. pH 5.5 |
| Water | q.s. to 100 ml | | |

Polyvinyl alcohol was slowly added to purified water, using around 80% of the desired batch volume, and allowed to hydrate during approximately 5 hours with continuous stirring. Then, the mixture was heated to 80°C with continuous mixing. Next, the solution was cooled to room temperature (between 22-25°C) with constant stirring. Then, the following ingredients were added slowly at room temperature with continuous mixing, waiting until each ingredient was dissolved before adding the next: sodium hyaluronate, NaH₂PO₄.H₂O, propylene glycol, polyethylene glycol 400, sodium chloride, and finally potassium sorbate. The pH was then adjusted with a 10% w/v solution of HCl, and the remaining amount of purified water was added. The solutions were independently prepared 3 times each, and the pH, osmolality, density, and viscosity were measured. Solution G was filtered through a 0.2 µm membrane to obtain an aseptic solution. The averages of the results for each solution are shown in the table below.

| **Parameter** | **Composition** | | |
|---|---|---|---|
| | **G** | **H** | **I** |
| **pH value** | 5.5 | 5.50 | 5.49 |
| **Osmolality (mOsm/kg)** | 258 | 238 | 227 |
| **Density (g/ml)** | 1.009 | 1.004 | 1.001 |
| **Viscosity (cSt)**** | 64.42 | 7.16 | 23.44 |

### EXAMPLE 4

A non randomized, double-masked, observational clinical trial is carried out in a single group of 36 patients to compare formulation G of Example 3 with Panoptic Lagrimas (Bausch & Lomb Argentina). Male and female patients from 21 to 70 years old with mild-to-moderate dry eye syndrome are recruited. Dry eye severity is assessed by recruited severity (mild or moderate KCS), patient self report (none, mild, moderate), clinician-report (none, mild, moderate) and quantitative measurement tests at each of the patient visit.

Each patient is treated with two drops a day for 30 days of formulation G of Example 3 in the right eye and Panoptic Lagrimas in the left eye. The following tests are carried out: visual acuity, Schirmer's test (without anesthesia), slit lamp tests, Texas Eye Research and Technology Center dry eye symptoms questionnaire (TERTC-DEQ), non-invasive break-up-time test, lissamine green staining, and fluorescein staining. The following pathologies are evaluated before and after treatment: corneal hyperemia, conjunctival hyperemia, meibomian gland dysfunction, pterygium, pinguecula, blepharitis, punctal phimosis, punctal occlusion, canalicular occlusion, corneal vascularization, corneal scars, corneal abrasion, and corneal ulcers. TERTC-DEQ is recorded at the baseline and 30 days. Symptoms and signs are recorded at the baseline, 7 days, 21 days, and 30 days.

For all the quantitative variables an analysis of variance (ANOVA) is used to detect differences between formulation G of Example 3 and Panoptic Lagrimas treatment groups. A statistically significant difference at the 95% confidence level is assumed in the presence of a p value <0.05.

### EXAMPLE 5

Ophthalmic solutions were prepared as described bellow containing the following ingredients in the amounts indicated in Table 4:

| **Table 4** | | | |
|---|---|---|---|
| **Ingredient** | **Composition (%w/v)** | | |
| | **J** | **K** | **L** |
| Dorzolamide hydrochloride *** | 2.23 | 2.23 | 2.23 |
| PVA | 2.0 | 2.0 | --- |
| Sodium hyaluronate | 0.2 | --- | 0.2 |
| NaH₂PO₄.H₂O | 0.05 | 0.05 | 0.05 |
| Propylene glycol | 1.0 | 1.0 | 1.0 |
| Polyethylene glycol 400 | 0.4 | 0.4 | 0.4 |
| NaCl | 0.1 | 0.1 | 0.1 |
| Potassium sorbate | 0.18 | 0.18 | 0.18 |
| NaOH | q.s. pH 5.5 | q.s. pH 5.5 | q.s. pH 5.5 |
| Water | q.s. to 100 ml | | |

| | | | |
|---|---|---|---|
| *** 2.23% w/v dorzolamide hydrochloride is equivalent to 2% w/v dorzolamide base. | | | |

Polyvinyl alcohol was slowly added to purified water, using around 80% of the desired batch volume, and allowed to hydrate during approximately 5 hours with continuous stirring. Then, the mixture was heated to 80°C with continuous mixing. Next, the solution was cooled to room temperature (between 22-25°C) with constant stirring. Then, the following ingredients were added slowly at room temperature with continuous mixing, waiting until each ingredient was dissolved before adding the next: sodium hyaluronate, NaH₂PO₄.H₂O, propylene glycol, polyethylene glycol 400, sodium chloride, potassium sorbate and dorzolamide hydrochloride. The pH was then adjusted with a 10% w/v solution of NaOH, and the remaining amount of purified water was added. The solutions were independently prepared 3 times each, and the pH, osmolality, density, and viscosity were measured. Solutions were filtered through a 0.2 µm membrane to obtain aseptic solutions. The averages of the results for each solution are shown in the table below.

| **Parameter** | **Composition** | | |
|---|---|---|---|
| | **J** | **K** | **L** |
| pH value | 5.51 | 5.53 | 5.50 |
| Osmolality (mOsm/kg) | 327 | 321 | 310 |
| Density (g/ml) | 1.014 | 1.012 | 1.008 |
| Viscosity (cSt)** | 53.62 | 6.73 | 14.10 |

The above is a detailed description of particular embodiments of the invention. It is recognized that departures from the disclosed embodiments may be made within the scope of the invention and that obvious modifications will occur to a person skilled in the art. Those of skill in the art should, in light of the present disclosure, appreciate that many changes can be made in the specific embodiments which are disclosed herein and still obtain a like or similar result without departing from the spirit and scope of the invention. All of the embodiments disclosed and claimed herein can be made and executed without undue experimentation in light of the present disclosure.

## Claims

1. An aqueous ophthalmic solution comprising a viscosity enhancing amount of the combination of 0.05 to 0.4 % w/v hyaluronic acid or a pharmaceutically acceptable salt thereof, and 0.25 to 4.0 % w/v polyvinyl alcohol, wherein the solution has a viscosity of 20 to 150 centistoke and is buffered to a pH 5.0 to 8.5.

2. The solution of claim 1, wherein the solution has less than 0.6 % w/v the one or more ionic-tonicity agents.

3. The solution of claims 1 or 2, wherein the pharmaceutically acceptable salt of hyaluronic acid is sodium hyaluronate.

4. The solution of claim 3, wherein the content of sodium hyaluronate ranges from 0.05 to 0.3 % w/v.

5. The solution of claim 4, wherein the content of sodium hyaluronate ranges from 0.1 to 0.3 % w/v.

6. The solution of claim 5, wherein the content of sodium hyaluronate is about 0.1 % w/v.

7. The solution of claim 5, wherein the content of sodium hyaluronate is about 0.2 % w/v.

8. The solution of claim any one of claims 1-7, wherein the content of polyvinyl alcohol ranges from 0.25 to 3 % w/v.

9. The solution of claim 8, wherein the content of polyvinyl alcohol is about 2 % w/v.

10. The solution of claims any one of claims 1-9, further comprising one or more pharmaceutically acceptable agents selected from the group consisting of buffering agents, preservative agents, preservative adjuncts, surfactants, solubilizing agents, stabilizing agents, comfort-enhancing agents, emollients, pH-adjusting agents, demulcents, lubricants, and mixtures thereof.

11. The solution of claim 10, wherein the buffering agent is selected from the group consisting of boric acid, dibasic sodium phosphate, monobasic sodium phosphate, hydrochloric acid, sodium hydroxide, tris(hydroxymethyl)aminomethane, bis(2-hydroxyethyl)iminotris-(hydroxymethyl)methane, sodium hydrogen carbonate, and mixtures thereof.

12. The solution of claim 11, wherein the content of monobasic sodium phosphate is about 0.05 % w/v.

13. The solution of claim 10, wherein the preservative agent is selected from the group consisting of benzalkonium chloride, per-salts, alcohols, peroxides, preservative agents containing quaternary ammonium salts, biguanide-contaning preservatives, chlorine dioxide precursors, metal chlorites, sorbic acid and ophthalmically acceptable salts thereof, and mixtures thereof.

14. The solution of claim 13, wherein the biguanide-contaning preservative is polyhexamethylene biguanide.

15. The solution of claim 14, wherein the content of polyhexamethylene biguanide is about 0.001 % w/v.

16. The solution of claim 13, wherein the preservative agent is benzalkonium chloride.

17. The solution of claim 16, wherein the content of benzalkonium chloride ranges from about 0.01 to about 0.10 % w/v.

18. The solution of claim 17, wherein the content of benzalkonium chloride is about 0.01 % w/v.

19. The solution of claim 13, wherein the ophthalmically acceptable salt of sorbic acid is potassium sorbate.

20. The solution of claim 19, wherein the content of potassium sorbate ranges from about 0.10 to about 0.20 % w/v.

21. The solution of claim 20, wherein the content of potassium sorbate is about 0.18 % w/v.

22. The solution of claim 1, further comprising a tonicity agent.

23. The solution of claim 22, wherein the tonicity agent is selected from the group consisting of inorganic salts, electrolytes, sodium chloride, potassium chloride, sodium phosphate, potassium phosphate, sodium, potassium sulfates, sodium and potassium bicarbonates, alkaline earth metal salts, mannitol, dextrose, glycerin, propylene glycol, and mixtures thereof.

24. The solution of claim 2, wherein the ionic-tonicity agent is sodium chloride.

25. The solution of claim 24, wherein the content of sodium chloride is about 0.38 % w/v.

26. The solution of claim 24, wherein the content of sodium chloride is about 0.1 % w/v.

27. The solution of claim 10, wherein the demulcent is selected from the group consisting of glycerin, propylene glycol, hydroxypropyl methyl cellulose, carboxymethyl cellulose, methyl cellulose, hydroxyethyl cellulose, polyvinyl pyrrolidone, polyethylene oxide, polyethylene glycol, polyacrylic acid, and mixtures thereof.

28. The solution of claim 27, wherein the demulcent is propylene glycol.

29. The solution of claim 28, wherein the content of propylene glycol is about 1.0 % w/v.

30. The solution of claim 10, wherein the lubricant is selected from the group consisting of polyethylene glycol, cellulose derivatives, dextran 70, gelatin, glycerin, polysorbate 80, propylene glycol, povidone, and mixtures thereof.

31. The solution of claim 30, wherein the lubricant is polyethylene glycol.

32. The solution of claim 31, wherein the content of polyethylene glycol is about 0.4 % w/v.

33. The solution of claims any one of claims 1-32, further comprising one or more active agents.

34. The solution of claim 33, wherein the active agent is selected from the group consisting of anti-glaucoma agent, anti-infective agent, anti-inflammatory agent, antihistamine, anti-allergic agent, decongestant, hormone, vitamin, growth factor, cytokine, mucin, surface stimulating drug, immunomodulator, immune response modifier, cytokine modifying agent, immunosuppressive agent, antineoplastic agent, anti-angiogenesis agent, eyelash growth stimulators, and combinations thereof.

35. The solution of claim 34, wherein the anti-glaucoma agent is a beta-blocker.

36. The solution of claim 35, wherein the beta-blocker is selected from the group consisting of timolol, betaxolol, levobetaxolol, carteolol, metipranolol, levobunolol, and mixtures thereof.

37. The solution of claim 34, wherein the anti-glaucoma agent is a miotic.

38. The solution of claim 37, wherein the miotic is pilocarpine.

39. The solution of claim 34, wherein the anti-glaucoma agent is a carbonic anhydrase inhibitor.

40. The solution of claim 39, wherein the carbonic anhydrase inhibitor is selected from the group consisting of dorzolamide, brinzolamide, and mixtures thereof.

41. The solution of claim 40, wherein the carbonic anhydrase inhibitor is dorzolamide hydrochloride.

42. The solution of claim 34, wherein the anti-glaucoma agent is a prostaglandin.

43. The solution of claim 42, wherein the prostaglandin is selected from the group consisting of travoprost, latanoprost, bimatoprost, and mixtures thereof.

44. The solution of claim 34, wherein the anti-glaucoma agent is a serotonergic.

45. The solution of claim 34, wherein the anti-glaucoma agent is a muscarinic.

46. The solution of claim 34, wherein the anti-glaucoma agent is a dopaminergic agonist.

47. The solution of claim 34, wherein the anti-glaucoma agent is an adrenergic agonist.

48. The solution of claim 47, wherein the adrenergic agonist is selected from the group consisting of apraclonidine, brimonidine, dipivefrin, and mixtures thereof.

49. The solution of claim 34, wherein the anti-infective agent is an antibiotic.

50. The solution of claim 49, wherein the antibiotic is selected from the group consisting of ciprofloxacin, ofloxacin, moxifloxacin, tobramycin, gentamicin, sulfacetamide, trimethoprim, polymyxin B, and mixtures thereof.

51. The solution of claim 34, wherein the anti-infective agent is an antimicrobial peptide.

52. The solution of claim 34, wherein the anti-infective agent is an antiviral.

53. The solution of claim 52, wherein the antiviral is trifluridine.

54. The solution of claim 34, wherein the anti-infective agent is an antiparasitic.

55. The solution of claim 34, wherein the anti-infective agent is an antifungal.

56. The solution of claim 34, wherein the anti-inflammatory agent is a non-steroidal steroidal anti-inflammatory agent.

57. The solution of claim 56, wherein the non-steroidal steroidal anti-inflammatory agent is selected from the group consisting of suprofen, flurbiprofen, diclofenac, ketorolac, and mixtures thereof.

58. The solution of claim 34, wherein the anti-inflammatory agent is a steroidal anti-inflammatory agent.

59. The solution of claim 58, wherein the steroidal anti-inflammatory agent is selected from the group consisting of rimexolone, tetrahydrocortisol, prednisolone, dexamethasone, and mixtures thereof.

60. The solution of claim 34, wherein the anti-allergic agent is selected from the group consisting of olopatadine, cromolyn, lodoxamide, emedastine, epinastine, and mixtures thereof.

61. The solution of claim 34, wherein the decongestant is selected from the group consisting of naphazoline, phenylephrine, and mixtures thereof.

62. The solution of claim 34 wherein the hormone is an androgen.

63. The solution of claim 34, wherein the growth factor is epidermal growth factor.

64. An ophthalmic solution comprising the following ingredients in the amounts indicated:
| **Ingredient** | **Composition (%w/v)** |
|---|---|
| PVA | 2.0 |
| Sodium hyaluronate | 0.2 |
| NaH₂PO₄.H₂O | 0.05 |
| Propylene glycol | 1.0 |
| Polyethylene glycol 400 | 0.4 |
| NaCl | 0.1 |
| Potassium sorbate | 0.18 |
| HCl | q.s. pH 5.5 |
| Water | q.s. to 100 ml |

65. A method for treating conditions selected from the group consisting of dry eye syndrome, keratitis sicca, xerophthalmia, keratoconjunctivitis sicca, ocular discomfort, rhinological allergic complications, Sjogren's syndrome, and Stevens Johnson syndrome comprising the step of administering a solution of any one of claims 1-32 or 64.

66. A method of lubricating an eye comprising the step of administering a solution of any one of claims 1-32 or 64.
